Europäisches Patentamt

European Patent Office

Office européen des brevets

(11) Numéro de publication: **0 494 016 B1**

## (12) FASCICULE DE BREVET EUROPEEN

(45) Date de publication de fascicule du brevet: **28.06.95**

(51) Int. Cl.⁶: **C07C 69/01**, C07C 67/10

(21) Numéro de dépôt: **91403520.9**

(22) Date de dépôt: **23.12.91**

Le dossier contient des informations techniques présentées postérieurement au dépôt de la demande et ne figurant pas dans le présent fascicule.

(54) **Procédé de transvinylation et son application.**

(30) Priorité: **04.01.91 FR 9100074**

(43) Date de publication de la demande:
**08.07.92 Bulletin 92/28**

(45) Mention de la délivrance du brevet:
**28.06.95 Bulletin 95/26**

(84) Etats contractants désignés:
**CH DE GB IT LI NL**

(56) Documents cités:

**TETRAHEDRON, vol. 28, janvier 1972, pages 233-238, Pergamon Press, Oxford, GB;J.E. McKEON et al.: "The palladium (II) catalyzed vinyl interchange reaction-II"**

(73) Titulaire: **SOCIETE FRANCAISE HOECHST TOUR ROUSSEL HOECHST**
**1 Terrasse Bellini**
**F-92800 Puteaux (FR)**

(72) Inventeur: **Vallejos, Jean-Claude**
**52, rue des Poissonniers**
**F-75018 Paris (FR)**
Inventeur: **Christidis, Yani**
**53, boulevard de la Villette**
**F-75019 Paris (FR)**

(74) Mandataire: **Rinuy, Santarelli**
**14, avenue de la Grande Armée**
**F-75017 Paris (FR)**

## Description

La présente invention concerne un procédé de transvinylation et son application.

Les réactions de transvinylation entre l'acétate de vinyle et un acide carboxylique sont couramment effectuées en présence d'un ou plusieurs catalyseurs. Comme catalyseurs, on a successivement proposé des sels mercuriques, des catalyseurs palladiés, en présence ou non de sels de métaux alcalins et/ou d'hydroxyde de potassium (brevet allemand n° 1127888, brevet européen n° 54.154, demandes de brevet japonais n° 53(78)-127410 et 53(78)-77005), des mélanges de sels de palladium, de cuivre et de métaux alcalins (demande de brevet japonais n° 55(80)-104221 et demande de brevet allemand n° 2823660), et enfin, des catalyseurs palladiés complexés avec des bases tertiaires particulières tels que le diacétato-(2,2′-bipyridyl) palladium II, 1, ou le diacétato-(phénanthroline-1,10) palladium II, 2, décrits par T.A. Stephenson et al, J. Chem. Soc. 1965, 3632-40, et utilisés comme catalyseurs de transvinylation par J.E. McKean et al, Tetrahedron, 1972, 28, 233-8.

Ces derniers catalyseurs, quoiqu'actifs dans les réactions de transvinylation sont toutefois peu stables à la chaleur : ils sont connus comme se décomposant vers 80° C et, en outre, leur préparation telle que décrite est difficile et/ou onéreuse.

Afin d'obvier à ces inconvénients d'une part, et d'obtenir un catalyseur actif et pour lequel en fin de réaction le palladium engagé est aisémment récupérable d'autre part, la demanderesse a découvert un procédé de transvinylation entre l'acétate de vinyle ou le propionate de vinyle et un acide carboxylique de formule générale (I)

RCOOH     (I)

dans laquelle R représente un radical alkyle, cycloalkyle, cycloalkylalkyle, aralkyle ou aromatique, substitués ou non, en présence d'un catalyseur palladié, caractérisé par le fait que ce catalyseur est obtenu in situ, en faisant réagir dans le milieu réactionnel un dérivé du palladium choisi dans le groupe constitué par l'acétate de palladium II, le nitrate de palladium II, l'hydroxyde de palladium II et le palladium déposé sur charbon, avec une amine tertiaire choisie dans le groupe constitué par la bipyridyl-2,2′, l'orthophénanthroline ou la tétraméthylènediamine, que la quantité de palladium utilisée, exprimée en atome-gramme de palladium II pour 100 moles d'acide carboxylique de formule générale (I) est comprise entre 0,005 et 1 et que la quantité d'amine est supérieure à 1 et inférieure à 10 moles par atome-gramme de palladium II utilisé.

Il peut sembler étonnant que l'on puisse utiliser dans le procédé selon l'invention du palladium métallique, déposé sur charbon, mais on a constaté que ledit charbon contenait en surface suffisamment de palladium II à l'état d'oxyde de palladium II pour pouvoir être actif.

Dans ce qui précède, par radical alkyle, l'on entend de préférence un radical alkyle linéaire ou ramifié renfermant de 1 à 18 atomes de carbone tel qu'un radical méthyle, éthyle, propyle, méthyléthyle, décyle, etc...

Par radical cycloalkyle l'on entend de préférence un radical renfermant de 3 à 6 atomes de carbone tel qu'un radical cyclopropyle ou cyclobutyle.

Par radical cycloalkylalkyle l'on entend de préférence un radical renfermant de 4 à 7 atomes de carbone tel qu'un radical cyclopropylméthyle.

Par radical aralkyle l'on entend de préférence un radical renfermant de 7 à 15 atomes de carbone tel qu'un radical benzyle ou phénéthyle.

Par radical aromatique l'on entend de préférence un radical renfermant de 4 à 6 atomes de carbone dont le noyau renferme 0, 1 ou 2 hétéroatomes, dans ce cas de préférence un atome de soufre ou d'azote, et on peut citer par exemple un radical phényle ou thiényle.

Lorsque les radicaux ci-dessus comportent des substituants, il s'agit de préférence de radicaux alkyle linéaires ou ramifiés renfermant de 1 à 6 atomes de carbone ou des radicaux alcoxy renfermant de 1 à 6 atomes de carbone.

Dans des conditions préférentielles de mise en oeuvre, le procédé ci-dessus décrit est réalisé :
- à l'ébullition, en présence d'un excès d'acétate de vinyle ou de propionate de vinyle,
- en présence de 0,02 à 0,1 atome-gramme de palladium II pour 100 moles d'acide carboxylique de formule générale (I) mis en oeuvre,
- en présence de 1,2 à 3 moles de bipyridyle-2,2′, d'orthophénanthroline ou de tétraméthyléthylènediamine par atome-gramme de palladium II mis en oeuvre,
- en présence d'un inhibiteur de polymérisation tel que la phénothiazine ; celle-ci peut par exemple être utilisée à la dose de 1 mmole pour 100 moles d'ester de vinyle mis en oeuvre,

2

- en suivant l'avancement de la réaction par la formation soit d'acide acétique soit d'acide propionique selon que l'on utilise l'acétate ou le propionate de vinyle,
- en présence d'un catalyseur obtenu en faisant réagir de l'hydroxyde de palladium II avec un excès d'orthophénanthroline.

L'acétate ou le nitrate de palladium sont des produits commerciaux ainsi que le palladium déposé sur charbon ; l'hydroxyde de palladium est préparé selon le brevet français n° 1403398.

Selon une variante du procédé selon l'invention, l'acétate de palladium, qui est un produit onéreux, peut être remplacé avantageusement par une quantité équivalente d'un produit de formule générale II

$$(R_1\text{-COO})_2 Pd \qquad (II)$$

dans laquelle $R_1$ est un radical alkyle en $C_6$-$C_{12}$, linéaire ou ramifié, tel que ledit radical $R_1$ correspond par exemple à l'acide hexanoïque, heptanoïque, octanoïque ou laurique, et le palladium est à l'état PdII.

Le produit de formule générale (II) peut être notamment obtenu à partir d'un tétrachloropalladate de métal alcalin par deux méthodes, soit en transformant le tétrachloropalladate de métal alcalin en hydoxyde de palladium II par action d'un hydroxyde de métal alcalin puis en le faisant réagir avec un acide de formule générale (III)

$$R_1 COOH \qquad (III)$$

où $R_1$ a la signignification donnée précédemment, soit en faisant réagir le tétrachloropalladate de métal alcalin avec le même sel de métal alcalin de l'acide de formule générale (III), puis dans l'une ou l'autre méthode en extrayant de ce milieu aqueux le produit de formule générale (III) correspondant avec un solvant organique non miscible à l'eau. La préparation de l'hydroxyde de palladium II est décrite notamment dans le brevet français n° 1403398. On a ainsi préparé selon l'une ou l'autre méthode, l'hexanoate de palladium II, l'heptanoate de palladium II, l'octanoate de palladium II, le laurate de palladium II.

Tous ces produits organométalliques sont très solubles dans des solvants aprotiques apolaires tels que le benzène, ce qui autorise leur isolement aisé de leur milieu de préparation aqueux.

Le procédé selon la présente invention est particulièrement avantageux pour préparer des esters de vinyle d'acides aliphatiques ou benzoïques tels que le paratertiobutylbenzoate de vinyle ou le laurate de vinyle.

Par ailleurs, le procédé selon l'invention peut être mis en oeuvre jusqu'à des températures de 100° C. A cette température, le catalyseur obtenu selon le procédé de l'invention reste stable et ne fournit pas du palladium métallique suceptible de se déposer plus ou moins solidement sur les parois du réacteur. Cette stabilité à la température est précieuse, car au cours de la réaction de transvinylation il se forme, soit de l'acide acétique lorsqu'on utilise l'acétate de vinyle, soit de l'acide propionique lorsqu'on utilise le propionate de vinyle, qui présentent à la pression ambiante des températures d'ébullition supérieures à 100° C. En outre, en fin de réaction, le palladium présent dans le milieu réactionnel peut être facilement récupéré, et recyclé après traitement. Cette propriété est particulièrement intéressante lors de la production industrielle de dérivés destinés à la pharmacie ou à la cosmétique mettant en oeuvre des quantités importantes de palladium car les teneurs en métaux lourds doivent être très faibles dans ces dérivés, compte tenu des exigences particulières à ces industries.

La récupération du palladium présent dans de milieu réactionnel peut s'effectuer en le précipitant dans ce milieu à l'état de complexe insoluble facilement isolé ensuite par filtration. Cette précipitation peut être réalisée notamment, par addition dans le mileu réactionnel, soit d'acide oxalique, soit d'un chlorure de tétraalkylammonium dans lequel les groupements alkyles sont en $C_1$-$C_8$ éventuellement substitués par un groupement phényle, soit d'une résine échangeuse d'anions à groupements chlorure d'ammonium quaternaires. Après isolement des complexes palladiés insolubles, on obtient un milieu réactionnel contenant moins de 5 ppm de palladium. De ce milieu on isole l'ester de vinyle cherché par des moyens connus en soi, tels que la distillation. Avantageusement, on utilise soit 2 moles de chlorure de tétraalkylammonium, soit 1 mole d'acide oxalique, soit enfin une quantité de résine échangeuse d'anions dont la capacité d'échange est supérieure à 2 équivalents d'ions chlorures par atome-gramme de palladium mis en oeuvre.

La présente demande a enfin pour objet l'application du procédé ci-dessus décrit à la préparation notamment du paratertiobutylbenzoate de vinyle, du laurate de vinyle et du benzoate de vinyle.

Les exemples suivants illustrent l'invention.

Dans le tableau I, RCOOH a la significaiton donnée précédemment, BP représente le bipyridyle-2,2' et OP représente l'orthophénanthroline. Les quantités d'acide carboxylique RCOOH et d'acétate de vinyle sont exprimées en moles, les quantités de catalyseur sont exprimées en $10^{-4}$ moles. Toutes les réactions de transvinylation ont été effectuées à l'ébullition, en présence de 5 mmoles de phénothiazine pour 100 moles d'acide RCOOH. Le rendement est exprimé par rapport à la théorie calculée à partir de l'acide RCOOH mis en oeuvre. Le taux de PdII dans les eaux mères, EM, est exprimé en ppm.

## EXEMPLES 1-4

On dissout 1,774 g (10 mmoles) de chlorure de palladium II dans 3,58 g d'acide chlorhydrique à 37 % soit 36 mmoles, puis on dilue cette solution avec 35 g d'eau. On introduit ensuite dans cette solution, sous agitation, en maintenant la température en dessous de 35° C, 13 g d'eau contenant en solution 2,8 g (70 mmoles) d'hydroxyde de sodium, puis en 15 minutes, à une température inférieure à 35° C, 8,8 g (50 mmoles) d'acide octanoïque. Le milieu réactionnel est ensuite abandonné 15 minutes à la température ambiante puis il est extrait deux fois avec 10 g de toluène.

Les phases organiques d'extraction sont ensuite lavées à l'eau, puis elles sont réunies et séchées sur sulfate de sodium anhydre. On obtient ainsi 26,5 g d'une solution toluénique contenant 4,2 ± 0,1 % de palladium II pour un titre théorique de 4,1 % à l'état d'octanoate de palladium II : $Pd(OCOC_7H_{15})_2$. Ce catalyseur est désigné $C_{8-1}$.

De la même façon, au départ d'acide hexanoïque, on prépare l'hexanoate de palladium II, désigné $C_{6-1}$, au départ d'acide heptanoïque on prépare l'heptanoate de palladium II désigné $C_{7-1}$.

## EXAMPLE 5

On dissout 1,774 g (10 mmoles) de chlorure de palladium II dans 3,58 g d'acide chlorhydrique à 37 %, soit 36 mmoles, puis la solution est diluée avec 35 g d'eau. Dans cette solution refroidie et maintenue à une température inférieure à 30° C, on introduit en 15 minutes 7,2 g d'une solution aqueuse contenant 1,44 g (36 mmoles) d'hydroxyde de sodium. On obtient ainsi une solution aqueuse de tétrachloropalladate de sodium. On introduit ensuite dans cette solution 4 g (20 mmoles) d'acide laurique dissous dans 46 g de toluène puis, en 10 minutes, à 30° C, sous agitation 4 g d'une solution aqueuse contenant 0,8 g (20 mmoles) d'hydroxyde de sodium. Après 3 heures d'agitation, on décante puis on lave la phase aqueuse avec 2 fois 15 g de toluène et ensuite les phases organiques sont réunies. On obtient ainsi environ 79 g d'une solution toluénique contenant du laurate de palladium II et présentant un titre de 1,28 % en palladium II (théorie 1,34 %). Ce catalyseur est désigné par la suite $C_{12-2}$.

De la même façon, on prépare des solutions toluéniques contenant de l'hexanoate de palladium II.

## EXEMPLE 6

On chauffe 12 heures au reflux une solution constituée par :
- 178 g (1 mole) d'acide paratertiobutylbenzoïque,
- 430 g (5 moles) d'acétate de vinyle,
- 0,21 g (0,93 mmole) d'acétate de palladium,
- 0,43 g (2,75 mmoles) de bipyridyle-2,2′,
- 0,01 g (0,05 mmole) de phénothiazine,

puis on refroidit la solution réactionnelle à 20° C. A cette température, on introduit sous agitation 0,12 g (0,95 mmole) d'acide oxalique cristallisé avec deux molécules d'eau puis on abandonne le milieu réactionnel deux heures sous agitation à 20° C. Le précipité formé est ensuite isolé par filtration et le filtrat est distillé sous pression réduite. On obtient ainsi 151,2 g (0,75 mole) de paratertiobutylbenzoate de vinyle distillant à 110 ± 3° C sous un vide de 6,6 mbar soit un rendement de 75 % de la théorie.

## EXEMPLES 7-21

En opérant selon l'exemple 6 au départ des matières premières et dans les conditions opératoires mentionnées dans le tableau I, on obtient avec les rendements indiqués les esters de vinyle des acides mentionnés dans le tableau I.

# TABLEAU I

| | 6 | 7 | 8 | 9 | 10 | 11 | 12 | 13 | 14 | 15 | 16 | 17 | 18 | 19 | 20 | 21 |
|---|---|---|---|---|---|---|---|---|---|---|---|---|---|---|---|---|
| Acétate de vinyle | 5 | 5 | 5 | 350 | 2,5 | 2,5 | 1,25 | 2,5 | 5 | 5 | 5 | 5 | 1,25 | 5 | 2,5 | 1,25 |
| RCOOH nature | AT | AL | AB | AT | AT | AL | AT | AB | AB | AB | AB | AB | AB | AB | AT | AT |
| quantité | 1 | 1 | 1 | 70 | 0,5 | 0,5 | 0,25 | 0,5 | 1 | 1 | 1 | 1 | 0,25 | 1 | 0,5 | 0,25 |
| Catalyseur Acétate de Pd II | 9 | 2,34 | 10 | 163,9 | | | | | | | | | | | | |
| Hydroxyde de Pd II | | | | | 1,15 | 1,15 | 1,15 | 1,15 | | | | | | | | |
| Autre catalyseur nature | | | | | | | | | $C_{8}\text{-}1$ | $C_{6}\text{-}1$ | $C_{7}\text{-}1$ | $C_{12}\text{-}2$ | B | A | C | D |
| quantité | | | | | | | | | 10 | 10 | 10 | 10 | 2,5 | 10 | 16,5 | 58,7 |
| BP | 27,5 | | | | | | 1,75 | | | | | | | | | |
| OP | | 3,5 | 15 | 246 | 1,75 | 1,75 | | 1,75 | 15 | 15 | 15 | 15 | 3,75 | 15 | 17,5 | 176,1 |
| durée (heure) | 12 | 20 | 6 | 24 | 20 | 20 | 15 | 20 | 6 | 6 | 6 | 6 | 6 | 6 | 20 | 24 |
| rendement (%) | 75 | 83 | 89 | 81 | 79 | 79,4 | 79,6 | 83,7 | 83,7 | 83,6 | 85,3 | 84,4 | 83,5 | 2 | 21 | 83,7 |

EP 0 494 016 B1

# Notes sur le tableau I

AT : acide paratertiobutylbenzoïque

AL : acide laurique

AB : acide benzoïque

A : chlorure de palladium II utilisé en présence de 10 moles d'acétate de sodium par mole de chlorure de palladium II.

B : nitrate de palladium II

C : oxyde de palladium II ; $PdO, H_2O$

D : Hydroxyde de palladium II préparé selon FR 1403398 titrant pondéralement 11,4 % de palladium II.

$C_{6^-1}$ : hexanoate de palladium II préparé à l'exemple 2

$C_{7^-1}$ : heptanoate de palladium II préparé à l'exemple 3

$C_{12^-2}$ : laurate de palladium II préparé à l'exemple 5

D : Palladium déposé sur charbon à 5 % en poids vendu par la Société JOHNSON MATHEY sous la référence 38 H.

EXEMPLE 22

On chauffe 24 heures au reflux un mélange de :
- 12,46 kg (70 moles) d'acide paratertiobutylbenzoïque,
- 30,1 kg (350 moles) d'acétate de vinyle,
- 3,68 g (16,4 mmoles) d'acétate de palladium II,
- 4,43 g (24,6 mmoles) d'orthophénanthroline,
- 0,7 g (3,5 mmoles) de phénothiazine,

puis on refroidit la solution réactionnelle à 20°C. A cette température, on introduit sous agitation 7,62 g (33,45 mmoles) de chlorure de triéthylbenzylammonium et on abandonne ensuite le milieu réactionnel 2 heures sous agitation à 20°C. Le précipité formé est ensuite isolé par filtration. On isole ainsi 6,2 g de complexe othophénanthroline - chlorure de palladium et dans le filtrat on dose 4 ppm de palladium II. Après traitement du filtrat, on obtient 11,5 kg (56,7 moles) de paratertiobutylbenzoate de vinyle distillant à 110 ± 3°C sous 6,6 mbar, soit un rendement de 81 % de la théorie calculée par rapport à l'acide paratertiobutylbenzoïque.

EXEMPLE 23

On reproduit l'exemple 22, mais en fin de réaction on remplace le chlorure de triéthylbenzylammonium utilisé par 0,89 g (7,06 mmoles) d'acide oxalique cristallisé avec deux molécules d'eau. On obtient ainsi après filtration du complexe formé, un filtrat contenant 3 ppm de palladium II.

EXEMPLE 24

On reproduit l'exemple 22 mais en fin de réaction on remplace le chlorure de triéthylbenzylammonium utilisé par 140 g de résine humide A26 commercialisée par la Société ROHM et HAAS à base de chlorure d'ammonium quaternaire. On obtient ainsi après filtration de la résine, un filtrat contenant 2 ppm de palladium II.

**Revendications**

1. Procédé de transvinylation entre l'acétate de vinyle ou le propionate de vinyle et un acide carboxylique de formule générale (I),

   RCOOH      (I)

   dans laquelle R représente un radical alkyle, cycloalkyle, aralkyle ou aromatique, substitués ou non, en présence d'un catalyseur palladié, caractérisé par le fait que ce catalyseur est obtenu in situ, en faisant réagir dans le milieu réactionnel un dérivé du palladium choisi dans le groupe constitué par l'acétate de palladium II, le nitrate de palladium II, l'hydroxyde de palladium II et le palladium déposé sur charbon, avec une amine tertiaire choisie dans le groupe constitué par le bipyridyl-2,2', l'orthophénanthroline, ou la tétraméthyléthylènediamine, que la quantité de palladium utilisée, exprimée en atome-gramme de palladium II pour 100 moles d'acide carboxylique de formule générale (I) est comprise entre 0,005 et 1 et que la quantité d'amine est supérieure à 1 et inférieure à 10 moles par atome-gramme de palladium II utilisé.

2. Procédé selon la revendication 1, caractérisé par le fait qu'il est effectué en présence de 0,02 à 0,1 atome-gramme de palladium II pour 100 moles d'acide carboxylique de formule générale (I) mis en oeuvre.

3. Procédé selon l'une quelconque des revendications 1 et 2, caractérisé par le fait qu'il est effectué en présence de 1,2 à 3 moles de bipyridyl-2,2', d'orthophénanthroline, ou de tétraméthyléthylènediamine par atome-gramme de palladium II utilisé.

4. Procédé selon l'une quelconque des revendications 1 à 3, caractérisé par le fait qu'il est effectué en présence d'un catalyseur obtenu en faisant réagir de l'hydroxyde de palladium II avec un excès d'orthophénanthroline.

5. Procédé selon l'une quelconque des revendications 1 à 3 caractérisé par le fait qu'il est effectué en présence d'acétate de palladium II.

6. Procédé selon la revendication 5, caractérisé par le fait que l'acétate de palladium est remplacé par une quantité équivalente d'un produit de formule générale (II)

   $(R_1\text{-COO})_2\ Pd$      (II)

   dans laquelle $R_1$ est un radical alkyle en $C_6$-$C_{12}$, linéaire ou ramifié.

7. Procédé selon la revendication 6, caractérisée par le fait que le produit de formule générale (II) est l'octanoate de palladium II.

8. Procédé selon l'une quelconque des revendications 1 à 7, caractérisé par le fait qu'en fin de réaction on récupère le palladium mis en oeuvre sous forme d'un complexe obtenu en le faisant réagir avec de l'acide oxalique.

9. Procédé selon l'une quelconque des revendications 1 à 7, caractérisé par le fait qu'en fin de réaction on récupère le palladium mis en oeuvre sous forme d'un complexe obtenu en le faisant réagir avec un chlorure de tétraalkylammonium dans lequel les groupements alkyles sont en $C_1$-$C_8$ éventuellement substitués par un groupement phényle.

10. Procédé selon l'une quelconque des revendications 1 à 7, caractérisé par le fait qu'en fin de réaction on récupère le palladium mis en oeuvre sous forme d'un complexe obtenu en le faisant réagir avec une résine échangeuse d'ions possédant des groupements chlorure d'ammonium quaternaires.

11. Application du procédé selon l'une quelconque des revendications 1 à 10 à la préparation du paratertiobutylbenzoate de vinyle.

12. Application du procédé selon l'une quelconque des revendications 1 à 10 à la préparation du laurate de vinyle.

13. Application du procédé selon l'une quelconque des revendications 1 à 10 à la préparation du benzoate de vinyle.

## Claims

1. Transvinylation process between vinyl acetate or vinyl propionate and a carboxylic acid of general formula (I),

   RCOOH   (I)

   in which R represents a substituted or non-substituted alkyl, cycloalkyl, aralkyl or aromatic radical, in the presence of a palladium catalyst, characterized by the fact that this catalyst is obtained in situ, by reacting, in the reaction medium, a derivative of palladium chosen from the group constituted by palladium II acetate, palladium II nitrate, palladium II hydroxide and palladium deposited on charcoal, with a tertiary amine chosen from the group constituted by 2,2'-bipyridyl, orthophenanthroline, or tetramethylethylenediamine, that the quantity of palladium used, expressed in gram-atom of palladium II per 100 moles of carboxylic acid of general formula (I) is comprised between 0.005 and 1 and that the quantity of amine is greater than 1 and less than 10 moles per gram-atom of palladium II used.

2. Process according to claim 1, characterized by the fact that it is carried out in the presence of 0.02 to 0.1 gram-atom of palladium II per 100 moles of carboxylic acid of general formula (I) employed.

3. Process according to any one of claims 1 and 2, characterized by the fact that it is carried out in the presence of 1.2 to 3 moles of 2,2'-bipyridyl, orthophenanthroline or tetramethylethylenediamine per gram-atom of palladium II used.

4. Process according to any one of claims 1 to 3, characterized by the fact that it is carried out in the presence of a catalyst obtained by reacting palladium II hydroxide with an excess of orthophenanthroline.

5. Process according to any one of claims 1 to 3, characterized by the fact that it is carried out in the presence of palladium II acetate.

6. Process according to claim 5, characterized by the fact that the palladium acetate is replaced by an equivalent quantity of a product of general formula (II)

   $(R_1-COO)_2Pd$   (II)

   in which $R_1$ is a linear or branched $C_6$-$C_{12}$ alkyl radical.

7. Process according to claim 6, characterized by the fact that the product of general formula (II) is palladium II octanoate.

8. Process according to any one of claims 1 to 7, characterized by the fact that at the end of the reaction the palladium employed is recovered in the form of complex which is obtained by reacting it with oxalic acid.

9. Process according to any one of claims 1 to 7, characterized by the fact that at the end of the reaction the palladium employed is recovered in the form of a complex which is obtained by reacting it with a tetraalkylammonium chloride in which the alkyl groups contain $C_1$-$C_8$ optionally substituted by a phenyl group.

10. Process according to any one of claims 1 to 7, characterized by the fact that at the end of the reaction the palladium employed is recovered in the form of a complex which is obtained by reacting it with an ion exchange resin having quaternary ammonium chloride groups.

11. Use of the process according to any one of claims 1 to 10 for the preparation of vinyl paratertiobutylbenzoate.

12. Use of the process according to any one of claims 1 to 10 for the preparation of vinyl laurate.

13. Use of the process according to any one of claims 1 to 10 for the preparation of vinyl benzoate.

**Patentansprüche**

1. Transvinylierungsverfahren zwischen Vinylacetat oder Vinylpropionat und einer Carbonsäure der allgemeinen Formel (I)

RCOOH (I)

in welcher R für einen substituierten oder unsubstituierten Alkyl-, Cycloalkyl-, Aralkylrest oder aromatischen Rest steht, in Gegenwart eines palladinierten Katalysators, dadurch gekennzeichnet, daß dieser Katalysator in situ erhalten ist, indem man im Reaktionsmedium ein Palladiumderivat, ausgewählt aus der aus Palladium(II)-acetat, Palladium(II)-nitrat, Palladium(II)-hydroxid und auf Kohle abgeschiedenem Palladium bestehenden Gruppe, mit einem tertiären Amin, ausgewählt aus der aus 2,2'-Bipyridyl, Orthophenanthrolin oder Tetramethylendiamin bestehenden Gruppe, umsetzt, so daß die verwendete Palladiummenge, ausgedrückt in Grammatom Palladium(II) auf 100 Mol Carbonsäure der allgemeinen Formel (I), zwischen 0,005 und 1 liegt und daß die Aminmenge über 1 und unter 10 Mol pro Grammatom verwendetem Palladium(II) liegt.

2. Verfahren nach Anspruch 1, dadurch gekennzeichnet, daß es in Gegenwart von 0,02 bis 0,1 Grammatom Palladium(II) auf 100 Mol verwendeter Carbonsäure der allgemeinen Formel (I) durchgeführt wird.

3. Verfahren nach einem der Ansprüche 1 und 2, dadurch gekennzeichnet, daß es in Gegenwart von 1,2 bis 3 Mol 2,2'-Bipyridyl, Orthophenanthrolin oder Tetramethylethylendiamin pro Grammatom verwendetem Palladium(II) durchgeführt wird.

4. Verfahren nach einem der Ansprüche 1 bis 3, dadurch gekennzeichnet, daß es in Gegenwart eines Katalysators durchgeführt wird, der erhalten worden ist, indem man Palladium(II)-hydroxid mit überschüssigem Orthophenanthrolin umsetzt.

5. Verfahren nach einem der Ansprüche 3 bis 3, dadurch gekennzeichnet, daß es in Gegenwart von Palladium(II)-acetat durchgeführt wird.

6. Verfahren nach Anspruch 5, dadurch gekennzeichnet, daß das Palladiumacetat durch einer äquivalente Menge eines Produktes der allgemeinen Formel (II)

$(R_1\text{-COO})_2$ Pd (II)

ersetzt wird, in welcher $R_1$ ein linearer oder verzweigter $C_6$-$C_{12}$-Alkylrest ist.

7. Verfahren nach Anspruch 6, dadurch gekennzeichnet, daß das Produkt der allgemeinen Formel (II) Palladium(II)-octanoat ist.

8. Verfahren nach einem der Ansprüche 1 bis 7, dadurch gekennzeichnet, daß man am Reaktionsende das verwendete Palladium in Form eines Komplexes zurückgewinnt, der durch Reaktion mit Oxalsäure erhalten worden ist.

9. Verfahren nach einem der Ansprüche 1 bis 7, dadurch gekennzeichnet, daß man am Reaktionsende das verwendete Palladium in Form eines Komplexes zurückgewinnt, der durch Reaktion mit einem Tetraalkylammoniumchlorid, in welchem die Alkylgruppen $C_1$-$C_8$-Alkylgruppen sind, die fakultativ mit einer Phenylgruppe substituiert sind, erhalten worden ist.

10. Verfahren nach einem der Ansprüche 1 bis 7, dadurch gekennzeichnet, daß man am Reaktionsende das verwendete Palladium in Form eines Komplexes zurückgewinnt, der durch Reaktion mit einem Ionenaustauscherharz, das quaternäre Ammoniumchloridgruppen aufweist, erhalten worden ist.

11. Anwendung des Verfahrens nach einem der Ansprüche 1 bis 10 zur Herstellung von p-tert-Butylbenzoesäurevinylester.

12. Anwendung des Verfahrens nach einem der Ansprüche 1 bis 10 zur Herstellung von Vinyllaurat.

13. Anwendung des Verfahrens nach einem der Ansprüche 1 bis 10 zur Herstellung von Vinylbenzoat.